# EUROPÄISCHE PATENTSCHRIFT

(11) **EP 1 318 160 B1**
(45) Veröffentlichungstag und Bekanntmachung des Hinweises auf die Patenterteilung: **26.09.2007**
(21) Anmeldenummer: 02022727.8
(22) Anmeldetag: 11.10.2002
(51) Int. Cl.: C08G 18/16, C08G 18/02, C07D 251/34

(54) **Katalysatoren und Verfahren zur Herstellung von Isocyanuratgruppen enthaltenden Polyisocyanaten und deren Verwendung**
Catalysts and process for the preparation of isocyanurate group containing polyisocyanates and their use
Catalyseurs et procédé pour la préparation de polyisocyanates contenant des groupes d'isocyanurate et leur utilisation

(30) Priorität: 05.12.2001 DE 10159803
(43) Veröffentlichungstag der Anmeldung: 11.06.2003
(73) Patentinhaber: Degussa GmbH, 40474 Düsseldorf (DE)
(72) Erfinder: Kohlstruk, Stephan, Dr., 48249 Dülmen (DE); Poersch, Waltraud, 44628 Herne (DE); Ewald, Michael, 45768 Marl (DE); Windmüller, Manuela, 45768 Marl (DE)

(56) Entgegenhaltungen:
- EP-A- 0 089 297
- EP-A- 0 447 074
- EP-A- 0 671 426

## Beschreibung

Die vorliegende Erfindung betrifft Katalysatoren und ein Verfahren zur Herstellung von Isocyanuratgruppen enthaltenden Polyisocyanaten, damit hergestellte Polyisocyanate und deren Verwendung.

Für hochwertige Ein- und Zweikomponenten Polyurethanlacke mit hoher Licht- und Wetterbeständigkeit werden als Isocyanatkomponente insbesondere Isocyanurat- und Uretdiongruppen enthaltende Polyisocyanatmischungen eingesetzt. Die Oligo- bzw. Polymerisierung von Isocyanaten zu derartigen Polyisocyanaten ist seit langem bekannt. Eine Reihe von Herstellverfahren wurde entwickelt, die sich in bezug auf die Katalysatorauswahl, in bezug auf die einzusetzenden organischen Isocyanate oder auch in Hinblick auf verfahrenstechnische Parameter voneinander unterscheiden (vgl. z. B. GB-PS 1391066, EP 82 987, DE 39 02 078, EP 339 396, EP 224 165; s. auch H.J. Laas et al. in J. Prakt. Chem. 336 (1994), 185ff.).

Zur Trimerisierung geeignete Isocyanate, z. B. aromatische, cycloaliphatische und aliphatische di- und höherwertige Polyisocyanate, können nach verschiedenartigen Verfahren hergestellt werden (Annalen der Chemie 562 (1949), Seiten 75ff). Technisch insbesondere bewährt hat sich die Herstellung durch Phosgenierung von organischen Polyaminen zu den entsprechenden Polycarbaminsäurechloriden und deren thermische Spaltung in organische Polyisocyanate und Chlorwasserstoff. Alternativ können organische Polyisocyanate auch ohne die Verwendung von Phosgen, d. h. nach phosgenfreien Verfahren, hergestellt werden. Nach Angaben der EP-B-0 126 299, EP 126 300 und EP 355 443 beispielsweise können (cyclo)aliphatische Diisocyanate - wie 1,6-Hexamethylendiisocyanat (HDI) und/oder isomere aliphatische Diisocyanate mit 6 Kohlenstoffatomen im Alkylenrest und 1-Isocyanato-3-isocyanato-methyl-3,5,5-trimethyl-cyclohexan (Isophorondiisocyanat bzw. IPDI) - hergestellt werden durch Umsetzung der zugrundeliegenden (cyclo)aliphatischen Diamine mit Harnstoff und Alkoholen zu (cyclo)aliphatischen Biscarbaminsäureestern und deren thermische Spaltung in die entsprechenden Diisocyanate und Alkohole.

Zur Oligomerisierung lässt man die (cyclo)aliphatischen Diisocyanate in Gegenwart des Katalysators, gegebenenfalls unter Verwendung von Lösungsmitteln und/oder Hilfsstoffen, bis zum Erreichen des gewünschten Umsatzes reagieren. Man spricht in diesem Zusammenhang auch von partieller Trimerisierung, da der angestrebte Umsatz in der Regel deutlich unterhalb von 100 % liegt. Danach wird die Reaktion durch Desaktivierung des Katalysators abgebrochen und das überschüssige monomere Diisocyanat üblicherweise abgetrennt, in der Regel durch Kurzweg- oder Dünnschichtdestillation. Die Desaktivierung erfolgt thermisch oder durch Zusatz eines Katalysatorinhibitors. Geeignet sind Säuren wie beispielsweise p-Toluolsulfonsäure oder Bis(2-Ethylhexyl)phosphat, Alkylierungsmittel oder auch Acylierungsreagenzien.

Als Katalysatoren für die Trimerisierung von Isocyanaten zu den angestrebten Isocyanurat- und ggf. Uretdiongruppen aufweisenden Polyisocyanaten können beispielsweise tertiäre Amine, Phosphine, Alkaliphenolate, Aminosilane, quartäre Ammoniumhydroxide oder quartäre Ammoniumcarbonate eingesetzt werden. Gut geeignete Oligomerisierungskatalysatoren sind auch Hydroxide, Halogenide oder Carboxylate von Hydroxyalkylammonium-Ionen (vgl. z. B. EP 351 873, EP 798 299, US 5 290 902), Alkalimetallsalze sowie Zinn-, Zink- bzw. Bleisalze von Alkylcarbonsäuren. In Abhängigkeit vom Kontakt ist auch die Verwendung von diversen Co-Katalysatoren wie z. B. OHfunktionalisierten Verbindungen oder Mannichbasen aus sekundären Aminen und Aldehyden bzw. Ketonen möglich.

Je nach verwendetem Katalysatortyp und Reaktionstemperatur erhält man Polyisocyanate mit unterschiedlichen Anteilen an Isocyanurat- bzw. Uretdiongruppen. Die Produkte sind meist klare, aber in Abhängigkeit vom Katalysatortyp, der Diisocyanatqualität, der Reaktionstemperatur und der Reaktionsfahrweise mehr oder weniger stark gelbgefärbte Produkte. Für die Herstellung hochwertiger Polyurethanlacke sind jedoch Produkte mit einer möglichst niedrigen Farbzahl erwünscht.

Die Auswahl eines geeigneten Katalysators kann nach unterschiedlichen Kriterien erfolgen.

Besonders vorteilhaft im Hinblick auf die Trimerisierung von Isocyanaten im technischen Maßstab ist zum Beispiel der Einsatz von quartärneren Hydroxyalkylammoniumcarboxylaten als Oligomerisierungskatalysatoren. Diese Kontakte vom Cholin-Typ sind thermisch labil. Es ist unnötig, die Trimerisierung bei Erreichen des gewünschten Umsatzes durch Zusatz potentiell qualitätsmindernder Katalysatorinhibitoren abzustoppen. Statt dessen erlaubt die gezielte thermische Deaktivierung eine optimale Prozesskontrolle. Vorteile bietet die Thermolabilität auch unter dem Gesichtspunkt der Prozesssicherheit. Ein unkontrolliertes "Durchgehen" der Reaktion ist ausgeschlossen, sofern die zudosierte Katalysatormenge das übliche Maß nicht um ein Vielfaches übersteigt.

Als vorteilhaft für die Herstellung von Isocyanuratgruppen enthaltenden Polyisocyanaten hoher Farbgüte haben sich Aminosilylverbindungen erwiesen (US 4 412 073, US 4 537 961, US 4 675 401, US 4 697 014). Darüber hinaus erlauben sie eine sichere Reaktionskontrolle und lassen sich mit Wasser oder Alkoholen leicht deaktivieren.

Die Klasse der Aminosilylkatalysatoren ist allerdings mit dem Nachteil einer geringen katalytischen Aktivität behaftet, so dass wirtschaftliche Raum-Zeit-Ausbeuten nur bei Einsatz verhältnismäßig großer Katalysatormengen realisiert werden können. Dies ist jedoch mit weiteren Nachteilen verbunden. Zum einen stellt es einen ernst zu nehmenden Kostenfaktor dar, denn der Kontakt wird durch die Deaktivierung irreversibel zerstört und kann nicht nutzbringend in den Prozess zurückgeführt werden. Zum anderen gelangen zwangsläufig größere Mengen des deaktivierten Katalysators in das Produkt mit ggf. negativen Konsequenzen für dessen Eigenschaftsprofil.

Es gibt daher einen Bedarf für Si-basierende Katalysatoren zur Herstellung von Isocyanuratgruppen enthaltenden Polyisocyanaten, die nicht die Nachteile der Aminosilylverbindungen des Stands der Technik aufweisen.

Der vorliegenden Erfindung lag demnach die Aufgabe zugrunde, neue Si-basierende Katalysatoren zur Herstellung von Isocyanuratgruppen enthaltenden Polyisocyanaten zur Verfügung zu stellen, die sich einerseits durch eine signifkant gesteigerte Aktivität auszeichnen, andererseits aber dennoch eine sichere Reaktionskontrolle und Prozesssicherheit gestatten.

Gegenstand der Erfindung ist ein Katalysator für die Trimerisierung von Isocyanaten, bestehend aus dem Reaktionsprodukt von
A) Verbindungen der allgemeinen Formel I

   R_{(4-q)}Si(NR¹R²)_{q} (I),

   bei der q = 1 oder 2 ist,
   R gleichzeitig oder unabhängig von einander für einen gesättigten oder ungesättigten, linearen oder verzweigten aliphatischen oder cycloaliphatischen Rest oder Aryl-, Aralkyl- oder Alkylarylrest mit 1 bis 16 C-Atomen steht und zwei Reste R über eine Alkylenbrücke miteinander verknüpft sein können,
   R¹ für R, SiR₃ oder einen Amidrest der Formel (II) 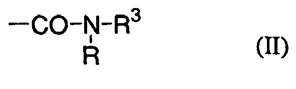 steht,
   R² gleich R oder H ist, wobei - sofern R¹ kein Amidrest ist - R² mit R¹ über eine Alkylenbrücke miteinander verknüpft sein kann, und
   R³ gleich R oder SiR₃ ist,
   und
B) Verbindungen ausgewählt aus Alkalifluoriden, Erdalkalifluoriden, Phosphazeniumfluoriden, Alkylarylaminoschwefeltrifluoriden, Dialkylaminoschwefeltrifluoriden, Diarylaminoschwefeltrifluoriden, Tetraalkylammonium-triphenyldifluorsilikate, Tetraalkylammonium-triphenyldifluorstannaten und Tetraalkylammoniumhexafluorosilikaten, Aminophosphoniumfluoriden der allgemeinen Formel (III) 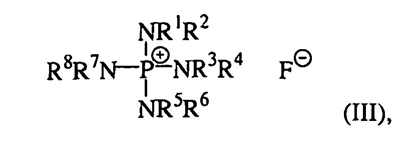
wobei R¹, R², R³, R⁴, R⁵, R⁶, R⁷ und R⁸ für verzweigte oder auch unverzweigte aliphatische, gegebenenfalls alkoxysubstituierte Alkylreste mit 1 bis 8 C-Atomen stehen und jeweils die Paare R¹ und R² sowie R³ und R⁴ sowie R⁵ und R⁶ sowie R⁷ und R⁸ über eine Alkylenbrücke, die gegebenenfalls auch die Heteroatome O,S oder N enthalten kann, miteinander verknüpft sein können,
im Verhältnis 1/100 < A/B < 100/1.

Gegenstand der Erfindung ist auch ein Verfahren zur Herstellung der erfindungsgemäßen Verbindungen und Katalysatoren. Dabei wird mindestens eine geeignete siliziumorganische Verbindung mit mindestens einer Si-N-Bindung (Verbindungen A) sowie mindestens ein geeignetes nucleophiles Fluorierungsmittel (Verbindungen B), gegebenenfalls in Gegenwart eines Solvatations- und/oder Komplexierungsmittels und/oder Phasentransferkatalysators, bei einer Temperatur von - 20 °C bis 200 °C miteinander zur Reaktion gebracht.

Zur Herstellung der erfindungsgemäßen Verbindungen und Katalysatoren geeignete Verbindungen A) sind Aminosilane, Silylharnstoffe oder Silazane oder auch Mischungen derselben, bespielsweise Methylaminotrimethylsilan, Diemthylaminotrimethylsilan, Dibutylaminotrimethylsilan, Diethylaminodimethylphenylsilan, Bis(dimethyamino)dimethylsilan, Bis(diethylamino)dimethylsilan, Bis(dibutylamino)dimethylsilan, Bis(dimethylamino)-methylphenylsilan, N-Methyl-N-trimethylsilyl-N'-methyl-N'-butylharnstoff, N-Trimethylsilyl-N-methyl-N',N'-dimethylharnstoff, N-Trimethylsilyl-N-ethyl-N',N'-dimethylharnstoff, N-Trimethylsilyl-N-butyl-N'-butyl-N'-trimethylsilylharnstoff, Trimethylsilylpyrrolidin, Trimethlsilylmorpholin, Trimethylsilylpiperidin, Trimethylsilylpiparazin, Hexamethyldisilazan, Heptamethyldisilazan, 1,3-Diethyl-1,1,3,3-tetramethyldisilazan, Hexaethyldisilazan und 1,3-Diphenyl-1,1,3,3-tetramethyldisilazan.

Zur Herstellung der erfindungsgemäßen Verbindungen und Katalysatoren geeignete Verbindungen B) sind Alkali- und Erdalkalifluoride beispielsweise Kaliumfluorid, Caesiumfluorid, Phosphazeniumfluoride wie zum Beispiel 1,1,1,3,3,3-Hexakis(dimethylamino)-diphosphazeniumfluorid (Lit.: R. Schwesinger et al., Angew. Chem. 103, 1991, 1376-1378), Aminophosphoniumfluoride der allgemeinen Formel (III) 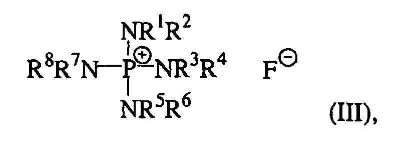 wobei R¹, R², R³, R⁴, R⁵, R⁶, R⁷ und R⁸ für verzweigte oder auch unverzweigte aliphatische, gegebenenfalls alkoxysubstituierte Alkylreste mit 1 bis 8 C-Atomen stehen und jeweils die Paare R¹ und R² sowie R³ und R⁴ sowie R⁵ und R⁶ sowie R⁷ und R⁸ über eine Alkylenbrücke, die gegebenenfalls auch die Heteroatome O,S oder N enthalten kann, miteinander verknüpft sein können, wie zum Beispiel Bis(2-methoxyethyl)amino-tris(pyrrolidino) phosphoniumfluorid, Bis(2-methoxyethyl)amino-tris(piperidino)phosphoniumfluorid, Bis(2-methoxyethyl)amino-tris(dimethylamino)phosphoniumfluorid oder Morpholino-tris (diethylamino)phosphoniumfluorid, Dialkyl-, Diaryl- oder Alkylarylaminoschwefeltrifluoride wie zum Beispiel Bis(2-methoxyethyl)aminoschwefeltrifluorid oder Diethylaminoschwefeltrifluorid sowie Tetraalkylammonium-triphenyldifluorsilikate, -triphenyldifluorstannate und -hexafluorosilikate, wie zum Beispiel Tetrabutylammoniumtriphenyldifluorsilikat, Tetrabutylammoniumtriphenyl-difluorstannat und Tetrabutylammoniumhexafluorosilikat.

Die Herstellung der erfindungsgemäßen Verbindungen und Katalysatoren kann "in situ" erfolgen, d.h. der Katalysator wird in der Di- oder Polyisocyanatmatrix erzeugt, deren Trimerisierung durchgeführt werden soll. Dazu werden die zur Ausbildung des Katalysators notwendigen Komponenten A und B unabhängig voneinander dem entsprechenden Di- oder Polyisocyanat zugemischt. Alternativ können die Katalysatorkomponenten zunächst außerhalb der Isocyanatmatrix in Substanz bzw. in einem Lösemittel miteinander zur Reaktion gebracht und der Kontakt auf diese Weise vorgefertigt werden. Die Herstellung kann grundsätzlich in Gegenwart eines oder mehrerer Solvatations- und/oder Komplexierungsmittel(s) und/oder Phasentransferkatalysator(s) erfolgen. Geeignete Komplexierungsmittel sind Polyethylenoxide, zum Beispiel Ethylengylkoldimethylether oder Polyethylenglykoldimethylether, Kronenether wie zum Beispiel Dibenzo-18-Krone-6 oder N,N'-Dibenzyl-4,13-diaza-18-Krone-6 oder auch Cryptanden wie zum Beispiel 1,10-Diaza-4,7,13,16,21-pentaoxabicyclo[8.8.5]tricosan. Geeignete Phasentransferkatalysatoren sind organische Ammonium- und Phosphoniumsalze, die in der Polyisocyanatmatrix löslich sind.

Gegenstand der Erfindung ist auch die Verwendung der erfindungsgemäßen Verbindungen und Katalysatoren zur Trimerisierung von Mono-, Di- oder Polyisocyanaten.

Gegenstand der Erfindung ist weiterhin ein Verfahren zur Herstellung von Isocyanuratgruppen enthaltenden Polyisocyanaten durch katalytisch induzierte Trimerisierung organischer Mono-, Di- oder Polyisocyanate, wobei als Trimerisierungskatalysatoren erfindungsgemäße Katalysatoren zum Einsatz gelangen.

Zur Herstellung der erfindungsgemäßen Polyisocyanate können alle bekannten aliphatischen, cycloaliphatischen, araliphatischen sowie aromatischen Mono-, Di- sowie Polyisocyanate eines NCO-Gehaltes von weniger als 70 Gewichtsprozent in reiner Form oder als beliebige Mischungen untereinander verwendet werden. Beispielhaft seien aufgeführt: Cyclohexandiisocyanate, Methylcyclohexandiisocyanate, Ethylcyclohexandiisocyanate, Propylcyclohexandiisocyanate, Methyl-diethyl-cyclohexandiisocyanate, Phenylendiisocyanate, Toluylendiisocyanate, Bis(isocyanatophenyl)methan, Propandiisocyanate, Butandiisocyanate, Pentandiisocyanate, Hexandiisocyanate (z. B. Hexamethylendiisocyanat (HDI) oder 1,5-Diisocyanato-2-methylpentan (MPDI)), Heptandiisocyanate, Octandiisocyanate, Nonandiisocyanate (z. B. 1,6-Diisocyanato-2,4,4-trimethylhexan und 1,6-Diisocyanato-2,2,4-trimethylhexan (TMDI)), Nonantriisocyanate (z. B. 4-Isocyanatomethyl-1,8-octandiisocyanat (TIN)), Dekandi- und triisocyanate, Undekandi- und -triisocyanate, Dodecandi- und -triisocyanate, Isophorondiisocyanat (IPDI), Bis(isocyanatomethylcyclohexyl)methan (H₁₂MDI), Isocyanatomethylmethylcyclohexylisocyanate, 2,5(2,6)-Bis(isocyanatomethyl)bicyclo[2.2.1]heptan (NBDI), 1,3-Bis(isocyanatomethyl)cyclohexan (1,3-H₆-XDI) sowie 1,4-Bis(isocyanatomethyl)cyclohexan (1,4-H₆-XDI). Die Aufzählung versteht sich inklusive aller Regio- und Stereoisomeren der beispielhaft benannten Isocyanate. Bevorzugt werden HDI, IPDI, MPDI, TMDI, 1,3- und 1,4- H₆-XDI, NBDI sowie Mischungen aus HDI und IPDI eingesetzt.

Auch Monoisocyanate können in Gegenwart der erfindungsgemäßen Katalysatoren in Isocyanurate überführt werden, zum Beispiel Ethylisocyanat, Cyclohexylisocyanat, Phenylisocyanat, Tolylisocyanat, Benzylisocyanat sowie alle Regio- und Stereoisomeren der nachstehend benannten Verbindungen: Propylisocyanate, Hexylisocyanate, Octylisocyanate und Methoxypropylisocyanat.

Für das erfindungsgemäße Verfahren ist es unwesentlich, über welchen Syntheseweg das eingesetzte Isocyanat hergestellt wurde, d. h mit oder ohne Verwendung von Phosgen. Es sei jedoch darauf hingewiesen, dass die zur Erreichung eines wunschgemäßen NCO-Gehaltes notwendige Katalysatormenge auch von der Qualität des Mono-, Di- oder Polyisocyanats abhängt. Erfahrungsgemäß macht ein ansteigender Gehalt des Isocyanats an hydrolysierbaren Chlorverbindungen eine Erhöhung der Katalysatormenge erforderlich, so dass ein inhibitiver Effekt des hydrolysierbaren Chlors auf den Kontakt angenommen werden kann.

Ebenso wie Aminosilylverbindungen können auch verschiedene nucleophile Fluorierungsmittel, unter ihnen beispielsweise Kaliumfluorid und Caesiumfluorid, die Trimerisierung von Isocyanaten induzieren (s. EP 0315 692; Y.Nambu, T.Endo, J. Org. Chem. 1993, 58, 1932-1934). Die aus Kombination dieser beiden Spezies hervorgehenden erfindungsgemäßen Katalysatoren weisen jedoch eine höhere Trimerisierungsaktivität auf. Sie können chemisch oder zum Teil auch thermisch deaktiviert werden.

Es ist zur Herstellung der erfindungsgemäßen Isocyanuratgruppen enthaltenden Polyisocyanate unwesentlich, ob der erfindungsgemäße Katalysator in dem zu trimerisierenden Mono-, Di- oder Polyisocyanat löslich ist, oder nicht.

Die Herstellung der Isocyanuratgruppen enthaltenden Polyisocyanate durch partielle Trimerisierung kann kontinuierlich (Rohrreaktor oder Kesselkaskade) erfolgen oder auch batchweise durchgeführt werden. Die erfindungsgemäßen Katalysatoren werden in geringer Konzentration zwischen 0.01 und 5.0 Gew.-%, eingesetzt. Die exakte Menge ist abhängig vom individuellen Katalysator, vom Umsatzziel und von der Verfahrensweise.

Die Trimersierung kann isotherm in einem Temperaturbereich zwischen 0 °C und 100 °C, bevorzugt 20 °C und 80 °C durchgeführt werden. Die Reaktion kann unter quantitativer Umsetzung der beteiligten Isocyanatgruppen des/der Ausgangs(poly)isocyanat(es)/mischung erfolgen oder bei beliebigen Umsetzungsgraden unterbrochen werden. Bevorzugt wird ein Umsatz von 10 - 50 % angestrebt. Ist der gewünschte Umsatz erreicht, wird die Trimerisierung durch Zudosierung (unter)stöchiometrischer Mengen eines Deaktivierungsmittels abgestoppt. Zur Inhibierung des Katalysatorsystems eignen sich zum Beispiel Säuren oder Säurederivate wie HCl, organische Sulfonsäuren oder saure Ester der phosphorigen Säure und Phosphorsäure.

Die Reaktionsführung kann auch exotherm ausgelegt werden. In diesem Fall wird die Temperatur der Reaktionsmischung bestehend aus dem erfindungsgemäßen Katalysator und dem Ausgangs(poly)isocyanat bzw. der Ausgangs(poly)isocyanat(mischung) zur Initiierung der exothermen Trimerisierung auf 120 - 160 °C, bevorzugt auf 80 - 120 °C, erhöht. Alternativ können die zur Bildung des erfindungsgemäßen Katalysators notwendigen Bestandteile bzw. der Katalysator in vorgefertigter Form auch zudosiert werden, nachdem das Ausgangs(poly)isocyanat bzw. die Ausgangs(poly)isocyanat(mischung) die zur Initiierung der exothermen Reaktion notwendige Temperatur erreicht hat. Die exakte Temperatur, bei der die exotherme Umsetzung initiiert wird, ist unter anderem eine Funktion des Isocyanats, des individuellen Katalysators und der Katalysatorkonzentration und kann leicht experimentell ermittelt werden. In aller Regel wird der erfindungsgemäße Katalysator im Zuge der exotherm verlaufenden Trimerisierung, bei der Temperaturen von bis zu 220 °C erreicht werden thermisch vollständig zerstört. Fortschreitender NCO-Verlust der Produkte der exothermen Trimerisierung bei Lagerung, z. B. bei 50 °C, weist darauf hin, dass die thermische Zerstörung des Katalysators nicht quantitativ erfolgt ist. In diesem Fall muss zur vollständigen Deaktivierung des Katalysators ein chemischer Inhibitor zugesetzt werden. Die notwendige Menge lässt sich leicht experimentell ermitteln.

Das erfindungsgemäße Verfahren kann sowohl lösemittelfrei als auch unter Verdünnung des zur Trimerisierung vorgesehenen Mono-, Di- oder Polyisocyanates bzw. der Mischungen davon durchgeführt werden. Zur Verdünnung eigenen sich grundsätzlich alle gegenüber NCO-Gruppen inerten organische Verbindungen wie z. B. Toluol, Xylol(e), höhere Aromaten, Ether und Ester. Die lösemittelfreie Variante ist bevorzugt.

Zur Herstellung Isocyanuratgruppen enthaltender Polyisocyanate werden die erfindungsgemäßen Katalysatoren der Formel (I) bevorzugt in Mengen von 0.01 - 5 Gew.-%, bevorzugt 0.02 - 3 Gew.-% , basierend auf dem Gewicht des eingesetzten Ausgangs(poly)isocyanates bzw. der Ausgangs(poly)isocyanat(mischung)(en), eingesetzt. Die exakte Menge lässt sich leicht experimentell ermitteln und ist abhängig von der katalytischen Aktivität des individuellen Kontaktes, dem Umsatzziel und der Verfahrensweise. Die Trimerisierung kann isotherm oder exotherm, kontinuierlich oder diskontinuierlich durchgeführt werden. Nach erfolgter chemischer oder thermischer Deaktivierung des Katalysators kann das nicht umgesetzte Monomer, sei es Mono-, Di- oder niedermolekulares Polyisocyanat, durch Kurzwegverdampfung, Dünnschichtverdampfung oder Extraktion abgetrennt und anschließend wiederverwendet werden. Die Abtrennung von überschüssigem/n Ausgangsisocyanat/en, erfolgt vorzugsweise, wenn die erfindungsgemäßen Verfahrensprodukte für Anwendungen im Polyurethanlack- und -beschichtungssektor bestimmt sind.

Gegenstand der Erfindung ist auch die Verwendung der monomerbefreiten isocyanuratgruppenhaltigen Polyisocyanate, als Zwischenprodukte für Polyurethanbeschichtungen, für Polyurethandispersionen, Klebstoffe und als Polyisocyanat-Komponente in 1- und 2- Komponenten Polyurethansystemen.

Die erfindungsgemäß hergestellten monomerbefreiten isocyanuratgruppenhaltigen Isocyanurate stellen nützliche Zwischenprodukte für Polyurethanbeschichtungen, z. B. Leder- und Textilbeschichtungen, und für Polyurethandispersionen und Klebstoffe dar, und sind besonders wertvoll als Polyisocyanat-Komponente in 1- und 2-Komponenten Polyurethansystemen für wetter- und lichtbeständige Polyurethanlacke. Dabei können die erfindungsgemäßen Verfahrenprodukte sowohl als solche als auch in mit Blockierungsmitteln blockierter Form zum Einsatz gelangen. Geeignete Blockierungsmittel sind dabei beispielsweise Lactame wie ε-Caprolactam, Oxime wie Methylethylketoxim oder Butanonoxim, Triazole wie 1H-1,2,4-Triazol, leicht enolisierbare Verbindungen wie Acetessigester oder Acetylaceton oder auch Malonsäurederivate wie Malonsäurediester mit 1-10 C-Atomen in den Alkoholresten.

### Beispiele

Alle Prozentangaben verstehen sich, sofern nicht anders angegeben, als Gewichtsprozente. Sämtliche Reaktionen wurden unter einer Stickstoffatmosphäre durchgeführt.

### Beispiel 1 (In situ-Herstellung des Katalysators und Trimerisierung)

a) Eine Mischung aus 800 g HDI, 1,2 g (0,15 %) Caesiumfluorid und 0,24 g (0,35 %) Heptamethyldisilazan wurde unter Rühren langsam auf 140 °C erhitzt. Nach 1 h ließ man auf 80°C abkühlen, versetzte zur Deaktivierung mit 0,12 g (0,018 %) Methanol und filtrierte vom Caesiumfluorid ab. Das Trimerisat wies einen NCO-Gehalt von 43,3 % auf (ca. 24 % Umsatz). Abschließend wurde das überschüssige Monomer vom Polyisocyanat durch Kurzwegverdampfung abgetrennt. Das entmonomerisierte Harz hatte einen NCO-Gehalt von 23,1 %.
b) Eine Mischung aus 800 g HDI und 2,4 ml einer 0,5 molaren Lösung 1,1,1,3,3,3-Hexakis(dimethylamino)-phosphazeniumfluorid und 1,4 g (0,18 %) Heptamethyldisilazan wurde vorsichtig auf 80 °C erhitzt. Nach 40 min betrug der NCO-Gehalt der Reaktionsmischung 40,2 % (ca. 36 % Umsatz). Die Reaktion wurde durch Zusatz von 5,8 g einer 2,5%igen Lösung von HCl in HDI abgestoppt, filtriert und überschüssiges Monomer durch Kurzwegverdampfung abgetrennt. Das monomerbefreite Polyisocyanat hatte einen NCO-Gehalt von 22,2 %.
c) Eine Mischung aus 800 g HDI und 0,7 g (0,08 %) Tetrabutylammonium-triphenyldifluorosilikat und 0,7 g (0,08 %) Heptamethyldisilazan wurde vorsichtig auf 60 °C erhitzt. Nach 90 min betrug der NCO-Gehalt der Reaktionsmischung 38,7 % (ca. 40 % Umsatz). Die Reaktion wurde durch Zusatz von 2,4 g einer 2,5%igen Lösung von HCl in HDI abgestoppt, filtriert und überschüssiges Monomer durch Kurzwegverdampfung abgetrennt. Das monomerbefreite Polyisocyanat hatte einen NCO-Gehalt von 21,9 %.

### Beispiel 2 (In situ-Herstellung des Katalysators und Trimerisierung)

a) Eine Mischung aus 800 g IPDI und 6,4 ml einer 0,5 molaren Lösung von 1,1,1,3,3,3-Hexakis(dimethylamino)-phosphazeniumfluorid und 1,4 g (0,18 %) Heptamethyldisilazan wurde vorsichtig auf 60 °C erhitzt. Nach 10 min bei 60 °C betrug der NCO-Gehalt der Reaktionsmischung 31,2 % (ca. 33 % Umsatz) . Die Reaktion wurde durch Zusatz von 10,1 g einer 2,9%igen Lösung von HCl in HDI abgestoppt, filtriert und überschüssiges Monomer durch Kurzwegverdampfung abgetrennt. Das monomerbefreite Polyisocyanat hatte einen NCO-Gehalt von 17,7 %.

### Beispiel 3 (Vergleichsbeispiel, nicht erfindungsgemäß)

a) Eine Mischung aus 1000 g HDI und 10 g (1 %) Heptamethyldisilazan wurde 2 h bei 140 °C gerührt. Anschließend ließ man auf Raumtemperatur abkühlen und bestimmte den NCO-Gehalt der Reaktionsmischung zu 38,6 % (entspricht einem Umsatz von ca. 40 %). Nach Deaktivierung des Katalysators mit 4 g Methanol wurde überschüssiges HDI durch Kurzwegverdampfung entfernt. Das monomerbefreite Polyisocyanat wies einen NCO-Gehalt von 21,8 % auf.
b) Eine Mischung aus 800 g HDI und 4 g (0,5 %) Heptamethyldisilazan wurde 4 h bei 140 °C gerührt. Anschließend ließ man auf Raumtemperatur abkühlen und bestimmte den NCO-Gehalt der Reaktionsmischung zu 40,1 % (entspricht einem Umsatz von ca. 36 %). Nach Deaktivierung des Katalysators mit 1,6 g Methanol wurde überschüssiges HDI durch Kurzwegverdampfung enfernt. Das monomerbefreite Polyisocyanat wies einen NCO-Gehalt von 22,0 % auf.
c) Eine Mischung aus 800 g HDI und 8 g (1 %) Heptamethyldisilazan wurde 8 h bei 100 °C gerührt. Anschließend ließ man auf Raumtemperatur abkühlen und bestimmte den NCO-Gehalt der Reaktionsmischung zu 39,9 % (entspricht einem Umsatz von ca. 36 %). Nach Deaktivierung des Katalysators mit 3,2 g Methanol wurde überschüssiges HDI durch Kurzwegverdampfung entfernt. Das monomerbefreite Polyisocyanat wies einen NCO-Gehalt von 21,9 % auf.

### Beispiel 4 (Vergleichsbeispiel, nicht erfindungsgemäß)

Eine Mischung aus 800 g IPDI und 8 g (1 %) Heptamethyldisilazan wurde 2 h bei 100 °C gerührt. Nachdem sich kein Umsatz feststellen ließ wurde weitere 2 h bei 140 °C gerührt. Der Umsatz betrug weniger als 3 %. Die Reaktion wurde abgebrochen, auf die Aufarbeitung wurde angesichts des geringfügigen Umsatzes verzichtet.

### Beispiel 5 (Vergleichsbeispiel, nicht erfindungsgemäß)

a) Eine Mischung aus 800 g HDI und 1,2 g (0,15 %) Caesiumfluorid wurde auf 100 °C aufgeheizt und 20 min bei dieser Temperatur belassen. Nachdem kein Umsatz festgestellt werden konnte, wurde die Temperatur weiter erhöht und die Reaktionsmischung 60 min bei 140 °C gerührt. Der Umsatz betrug weniger als 5 %. Die Reaktion wurde abgebrochen, auf die Aufarbeitung wurde angesichts des geringfügigen Umsatzes verzichtet.
b) Eine Mischung aus 800 g HDI und 7 ml einer 0,5 molaren Lösung von 1,1,1,3,3,3-Hexakis(dimethylamino)-phosphazeniumfluorid wurde vorsichtig auf 80 °C erhitzt. Nach 30 min betrug der NCO-Gehalt der Reaktionsmischung 40,1 % (ca. 36 % Umsatz). Die Reaktion wurde durch Zusatz von 5,1 g einer 2,5%igen Lösung von HCl in HDI abgestoppt und überschüssiges Monomer durch Kurzwegverdampfung abgetrennt. Das monomerbefreite Polyisocyanat hatte einen NCO-Gehalt von 22,4 %.

## Patentansprüche

1. Katalysator für die Trimerisierung von Isocyanaten, bestehend aus dem Reaktionsprodukt von
A) Verbindungen der allgemeinen Formel I
R_{(4-q)}Si(NR¹R²)_{q} (I),
bei der q = 1 oder 2 ist,
R gleichzeitig oder unabhängig von einander für einen gesättigten oder ungesättigten, linearen oder verzweigten aliphatischen oder cycloaliphatischen Rest oder Aryl-, Aralkyl- oder Alkylarylrest mit 1 bis 16 C-Atomen steht und zwei Reste R über eine Alkylenbrücke miteinander verknüpft sein können,
R¹ für R, SiR₃ oder einen Amidrest der Formel (II) 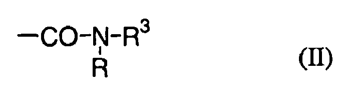 steht,
R² gleich R oder H ist, wobei - sofern R¹ kein Amidrest ist - R² mit R¹ über eine Alkylenbrücke miteinander verknüpft sein kann, und
R³ gleich R oder SiR₃ ist,
und
B) Verbindungen ausgewählt aus Alkalifluoriden, Erdalkalifluoriden, Phosphazeniumfluoriden, Alkylarylaminoschwefeltrifluoriden, Dialkylaminoschwefeltrifluoriden, Diarylaminoschwefeltrifluoriden, Tetraalkylammonium-triphenyldifluorsilikate, Tetraalkylammonium-triphenyldifluorstannaten und Tetraalkylammoniumhexafluorosilikaten, Aminophosphoniumfluoriden der allgemeinen Formel (III) 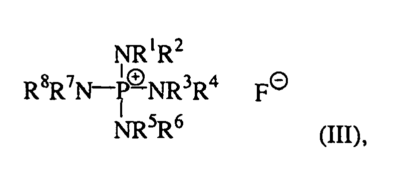
wobei R¹, R², R³, R⁴, R⁵, R⁶, R⁷ und R⁸ für verzweigte oder unverzweigte aliphatische, gegebenenfalls alkoxysubstituierte Alkylreste mit 1 bis 8 C-Atomen stehen und jeweils die Paare R¹ und R² sowie R³ und R⁴ sowie R⁵ und R⁶ sowie R⁷ und R⁸ über eine Alkylenbrücke, die gegebenenfalls auch die Heteroatome O, S oder N enthalten kann, miteinander verknüpft sein können,
im Verhältnis 1/100 < A/B < 100/1.

2. Katalysator nach Anspruch 1,
**dadurch gekennzeichnet,**
**dass** Aminosilane, Silylharnstoffe und/oder Silazane als Ausgangskomponenten A) enthalten sind.

3. Katalysator nach mindestens einem der Ansprüche 1 bis 2,
**dadurch gekennzeichnet,**
**dass** als Ausgangskomponenten A) Methylaminotrimethylsilan, Diemthylaminotrimethylsilan, Dibutylaminotrimethylsilan, Diethylaminodimethylphenylsilan, Bis(dimethyamino) dimethylsilan, Bis(diethylamino)dimethylsilan, Bis(dibutylamino)dimethylsilan, Bis (dimethylamino)methylphenylsilan, N-Methyl-N-trimethylsilyl-N'-methyl-N'-butylharnstoff, N-Trimethylsilyl-N-methyl-N',N'-dimethylharnstoff, N-Trimethylsilyl-N-ethyl-N',N'-dimethylharnstoff, N-Trimethylsilyl-N-butyl-N'-butyl-N'-trimethylsilylharnstoff, Trimethylsilylpyrrolidin, Trimethlsilylmorpholin, Trimethylsilylpiperidin, Trimethylsilyl-piparazin, Hexamethyldisilazan, Heptamethyldisilazan, 1,3-Diethyl-1,1,3,3-tetramethyldisilazan, Hexaethyldisilazan und oder 1,3-Diphenyl-1,1,3,3-tetramethyldisilazan enthalten sind.

4. Katalysator nach mindestens einem der Ansprüche 1 bis 3,
**dadurch gekennzeichnet,**
**dass** Kaliumfluorid, Caesiumfluorid, 1,1,1,3,3,3-Hexakis(dimethylamino)-diphosphazeniumfluorid als Ausgangskomponenten B) enthalten sind.

5. Katalysator nach mindestens einem der Ansprüche 1 bis 3,
**dadurch gekennzeichnet,**
**dass** als Ausgangsverbindungen B) Bis(2-methoxyethyl)amino-tris(pyrrolidino) phosphoniumfluorid, Bis(2-methoxyethyl)amino-tris(piperidino)phosphoniumfluorid, Bis (2-methoxyethyl)amino-tris(dimethylamino)phosphoniumfluorid, Morpholino-tris (diethylamino)phosphoniumfluorid, Bis(2-methoxyethyl)aminoschwefeltrifluorid, Diethylaminoschwefeltrifluorid, Tetrabutylammoniumtriphenyldifluorsilikat, Tetrabutylammoniumtriphenyldifluorstannat, Tetrabutylammoniumhexafluorsilikat enthalten sind.

6. Verfahren zur Herstellung einer Verbindung, bestehend aus dem Reaktionsprodukt von
A) Verbindungen der allgemeinen Formel I
R_{(4-q)}Si(NR¹R²)_{q} (I),
bei der q = 1 oder 2 ist,
R gleichzeitig oder unabhängig von einander für einen gesättigten oder ungesättigten, linearen oder verzweigten aliphatischen oder cycloaliphatischen Rest oder Aryl-, Aralkyl- oder Alkylarylrest mit 1 bis 16 C-Atomen steht und zwei Reste R über eine Alkylenbrücke miteinander verknüpft sein können,
R¹ für R, SiR₃ oder einen Amidrest der Formel (II) 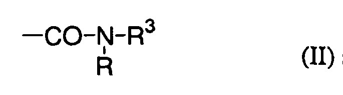 steht,
R² gleich R oder H ist, wobei - sofern R¹ kein Amidrest ist - R² mit R¹ über eine Alkylenbrücke miteinander verknüpft sein kann, und
R³ gleich R oder SiR₃ ist,
und
B) Verbindungen ausgewählt aus Alkalifluoriden, Erdalkalifluoriden, Phosphazeniumfluoriden, Alkylarylaminoschwefeltrifluoriden, Dialkylaminoschwefeltrifluoriden, Diarylaminoschwefeltrifluoriden, Tetraalkylammonium-triphenyldifluorsilikate, Tetraalkylammonium-triphenyldifluorstannaten und Tetraalkylammoniumhexafluorosilikaten, Aminophosphoniumfluoriden der allgemeinen Formel (III) 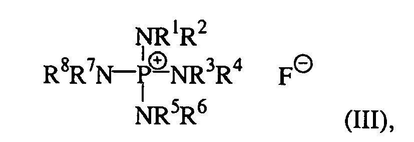
wobei R¹, R², R³, R⁴, R⁵, R⁶, R⁷ und R⁸ für verzweigte oder auch unverzweigte aliphatische, gegebenenfalls alkoxysubstituierte Alkylreste mit 1 bis 8 C-Atomen stehen und jeweils die Paare R¹ und R² sowie R³ und R⁴ sowie R⁵ und R⁶ sowie R⁷ und R⁸ über eine Alkylenbrücke, die gegebenenfalls auch die Heteroatome O,S oder N enthalten kann, miteinander verknüpft sein können, im Verhältnis 1/100 < AB < 100/1,
gegebenenfalls in Gegenwart eines Solvatations- und/oder Komplexierungsmittels und/oder Phasentransferkatalysators, bei einer Temperatur von- 20 °C bis 200 °C.

7. Verfahren nach Anspruch 6,
**dadurch gekennzeichnet,**
**dass** Ausgangsverbindungen gemäß den Ansprüchen 2 oder 3 und 4 oder 5 eingesetzt werden.

8. Verwendung der Verbindungen nach einem der Ansprüche 1 bis 6 zur Trimerisierung von Mono-, Di- oder Polyisocyanaten.

9. Verwendung nach Anspruch 8,
**dadurch gekennzeichnet,**
**dass** als Isocynatverbindungen aliphatische, cycloaliphatische, araliphatische und/oder aromatische Mono-, Di- oder Polyisocyanate eingesetzt werden.

10. Verwendung nach Anspruch 9,
**dadurch gekennzeichnet,**
**dass** Cyclohexandiisocyanate, Methylcyclohexandiisocyanate, Ethylcyclohexandiisocyanate, Propylcyclohexandiisocyanate, Methyl-diethyl-cyclohexandiisocyanate, Phenylendiisocyanate, Toluylendiisocyanate, Bis(isocyanatophenyl)methan, Propandiisocyanate, Butandiisocyanate, Pentandiisocyanate, Hexandiisocyanate, wie Hexamethylendiisocyanat (HDI) oder 1,5-Diisocyanato-2-methylpentan (MPDI), Heptandiisocyanate, Octandiisocyanate, Nonandiisocyanate, wie 1,6-Diisocyanato-2,4,4-trimethylhexan und 1,6-Diisocyanato-2,2,4-trimethylhexan (TMDI), Nonantriisocyanate, wie 4-Isocyanatomethyl-1,8-octandiisocyanat (TIN), Dekandi- und triisocyanate, Undekandi- und -triisocyanate, Dodecandi- und -triisocyanate, Isophorondiisocyanat (IPDI), Bis(isocyanatomethylcyclohexyl)methan (H₁₂MDI), Isocyanatomethyl-methylcyclohexylisocyanate, 2,5(2,6)-Bis(isocyanatomethyl)bicyclo[2.2.1]heptan (NBDI), 1,3-Bis(isocyanatomethyl)cyclohexan (1,3-H₆-XDI) oder 1,4-Bis(isocyanatomethyl) cyclohexan (1,4-H₆-XDI) eingesetzt werden.

11. Verwendung nach Anspruch 9,
**dadurch gekennzeichnet,**
**dass** HDI, IPDI, MPDI, TMDI, 1,3- und 1,4- H₆-XDI, NBDI oder Mischungen aus HDI und IPDI eingesetzt werden.

12. Verwendung nach Anspruch 9,
**dadurch gekennzeichnet,**
**dass** als Monoisocyanate Ethylisocyanat, Cyclohexylisocyanat, Phenylisocyanat, Tolylisocyanat, Benzylisocyanat sowie alle Regio- und Stereoisomeren der Verbindungen: Propylisocyanate, Hexylisocyanate, Octylisocyanate und Methoxypropylisocyanat eingesetzt werden.

13. Verfahren zur Herstellung von Isocyanuratgruppen enthaltenden Polyisocyanaten durch katalytisch induzierte Trimerisierung organischer Mono-, Di- oder Polyisocyanate, **dadurch gekennzeichnet, dass** Trimerisierungskatalysatoren, bestehend aus dem Reaktionsprodukt von
A) Verbindungen der allgemeinen Formel I
R_{(4-q)}Si(NR¹R²)_{q} (I),
bei der q = 1 oder 2 ist,
R gleichzeitig oder unabhängig von einander für einen gesättigten oder ungesättigten, linearen oder verzweigten aliphatischen oder cycloaliphatischen Rest oder Aryl-, Aralkyl- oder Alkylarylrest mit 1 bis 16 C-Atomen steht und zwei Reste R über eine Alkylenbrücke miteinander verknüpft sein können,
R¹ für R, SiR₃ oder einen Amidrest der Formel (II) 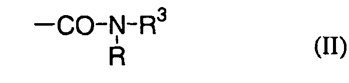 steht,
R² gleich R oder H ist, wobei - sofern R¹ kein Amidrest ist - R² mit R¹ über eine Alkylenbrücke miteinander verknüpft sein kann, und
R³ gleich R oder SiR₃ ist,
und
B) Verbindungen ausgewählt aus Alkalifluoriden, Erdalkalifluoriden, Phosphazeniumfluoriden, Alkylarylaminoschwefeltrifluoriden, Dialkylaminoschwefeltrifluoriden, Diarylaminoschwefeltrifluoriden, Tetraalkylammonium-triphenyldifluorsilikate, Tetraalkylammonium-triphenyldifluorstannaten und Tetraalkylammoniumhexafluorosilikaten, Aminophosphoniumfluoriden der allgemeinen Formel (III) 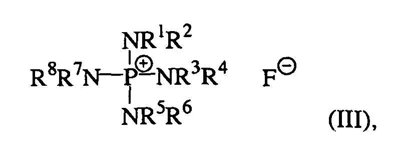
wobei R¹, R², R³, R⁴, R⁵, R⁶, R⁷ und R⁸ für verzweigte oder auch unverzweigte aliphatische, gegebenenfalls alkoxysubstituierte Alkylreste mit 1 bis 8 C-Atomen stehen und jeweils die Paare R¹ und R² sowie R³ und R⁴ sowie R⁵ und R⁶ sowie R⁷ und R⁸ über eine Alkylenbrücke, die gegebenenfalls auch die Heteroatome O,S oder N enthalten kann, miteinander verknüpft sein können,
im Verhältnis 1/100 < A/B < 100/1 eingesetzt werden.

14. Verfahren nach Anspruch 13,
**dadurch gekennzeichnet,**
**dass** aliphatische, cycloaliphatische, araliphatische, oder aromatische Mono-, Di oder Polyisocyanate eingesetzt werden.

15. Verfahren nach Anspruch 14,
**dadurch gekennzeichnet,**
**dass** Cyclohexandiisocyanate, Methylcyclohexandiisocyanate, Ethylcyclohexandiisocyanate, Propylcyclohexandiisocyanate, Methyl-diethyl-cyclohexandiisocyanate, Phenylendiisocyanate, Toluylendiisocyanate, Bis(isocyanatophenyl)methan, Propandiisocyanate, Butandiisocyanate, Pentandiisocyanate, Hexandiisocyanate, wie Hexamethylendiisocyanat (HDI) oder 1,5-Diisocyanato-2-methylpentan (MPDI), Heptandiisocyanate, Octandiisocyanate, Nonandiisocyanate, wie 1,6-Diisocyanato-2,4,4-trimethylhexan und 1,6-Diisocyanato-2,2,4-trimethylhexan (TMDI), Nonantriisocyanate, wie 4-Isocyanatomethyl-1,8-octandiisocyanat (TIN), Dekandi- und triisocyanate, Undekandi- und -triisocyanate, Dodecandi- und -triisocyanate, Isophorondiisocyanat (IPDI), Bis(isocyanatomethylcyclohexyl)methan (H₁₂MDI), Isocyanatomethyl-methylcyclohexylisocyanate, 2,5(2,6)-Bis(isocyanatomethyl)bicyclo[2.2.1]heptan (NBDI), 1,3-Bis(isocyanatomethyl)cyclohexan (1,3-H₆-XDI) oder 1,4-Bis(isocyanatomethyl) cyclohexan (1,4-H₆-XDI) eingesetzt werden.

16. Verfahren nach Anspruch 14,
**dadurch gekennzeichnet,**
**dass** HDI, IPDI, MPDI, TMDI, 1,3- und 1,4- H6-XDI, NBDI sowie Mischungen aus HDI und IPDI eingesetzt werden.

17. Verfahren nach Anspruch 14,
**dadurch gekennzeichnet,**
**dass** Monoisocyanate ausgewählt aus Ethylisocyanat, Cyclohexylisocyanat, Phenylisocyanat, Tolylisocyanat, Benzylisocyanat sowie alle Regio- und Stereoisomeren der Verbindungen Propylisocyanate, Hexylisocyanate, Octylisocyanate und Methoxypropylisocyanat eingesetzt werden.

18. Verfahren nach mindestens einem der Ansprüche 13 bis 17,
**dadurch gekennzeichnet,**
**dass** die Herstellung der Isocyanuratgruppen enthaltenden Polyisocyanate kontinuierlich oder batchweise durchgeführt wird

19. Verfahren nach mindestens einem der Ansprüche 13 bis 18,
**dadurch gekennzeichnet,**
**dass** die Katalysatoren in Konzentration zwischen 0.01 und 5.0 Gew.-%, eingesetzt werden.

20. Verfahren nach mindestens einem der Ansprüche 13 bis 19,
**dadurch gekennzeichnet,**
**dass** die Herstellung isotherm in einem Temperaturbereich zwischen 0 °C und 100 °C, bevorzugt 20 °C und 80 °C, durchgeführt wird.

21. Verwendung der monomerbefreiten isocyanuratgruppenhaltigen Polyisocyanate, hergestellt nach mindestens einem der Ansprüche 13 bis 20, als Zwischenprodukte für Polyurethanbeschichtungen, Polyurethandispersionen, Klebstoffe und als Polyisocyanat-Komponente in 1- und 2-Komponenten Polyurethansystemen.

22. Verwendung nach Anspruch 21 für Leder- und Textilbeschichtungen oder Polyurethanlacke.

## Claims

1. Catalyst for the trimerization of isocyanates, comprising the reaction product of
A) compounds of the general formula I
R_{(4-q)}Si(NR¹R²)_{q} (I),
in which q = 1 or 2,
R simultaneously or independently of one another stands for a saturated or unsaturated, linear or branched aliphatic or cycloaliphatic radical or aryl, aralkyl, or alkylaryl radical having from 1 to 16 carbon atoms, and two radicals R can be linked with one another via an alkylene bridge,
R¹ represents R, SiR₃ or an amide radical of formula (II) 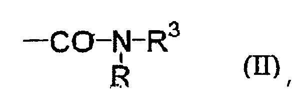 R² is R or H, it being possible for R², if R¹ is not an amide radical, to be linked to R¹ via an alkylene bridge,
R³ is R or SiR₃,
and
B) compounds selected from alkali metal fluorides, alkaline earth metal fluorides, phosphazenium fluorides, alkylarylaminosulfur trifluorides, dialkylaminosulfur trifluorides, diarylaminosulfur trifluorides, tetraalkylammonium triphenyldifluorosilicates, tetraalkylammonium triphenyldifluorostannates, and tetraalkylammonium hexafluorosilicates, aminophosphonium fluorides of the general formula (III) 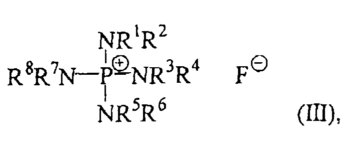
where R¹, R², R³, R⁴, R⁵, R⁶, R⁷, and R⁸ are branched or unbranched aliphatic, optionally alkoxy-substituted alkyl radicals having from 1 to 8 carbon atoms and where in each case the pairings R¹ and R² and also R³ and R⁴ and also R⁵ and R⁶ and also R⁷ and R⁸ may be linked to one another via an alkylene bridge, which where appropriate may also contain the heteroatoms O, S or N,
in a ratio 1/100 < A/B < 100/1.

2. Catalyst according to Claim 1, **characterized in that** amino silanes, silyl ureas and/or silazanes are present as starting components A).

3. Catalyst according to at least one of Claims 1 to 2,
**characterized in that** methylaminotrimethylsilane, dimethylaminotrimethylsilane, dibutylaminotrimethylsilane, diethylaminodimethylphenylsilane, bis(dimethylamino)dimethylsilane, bis(diethylamino)dimethylsilane, bis(dibutylamino)dimethylsilane, bis(dimethylamino)methylphenylsilane, N-methyl-N-trimethylsilyl-N'-methyl-N'-butylurea, N-trimethylsilyl-N-methyl-N',N'-dimethylurea, N-trimethylsilyl-N-ethyl-N',N'-dimethylurea, N-trimethylsilyl-N-butyl-N'-butyl-N'-trimethylsilylurea, trimethylsilylpyrrolidine, trimethylsilylmorpholine, trimethylsilyl-piperidine, trimethylsilylpiperazine, hexamethyldisilazane, heptamethyldisilazane, 1,3-diethyl-1,1,3,3-tetramethyldisilazane, hexaethyldisilazane and/or 1,3-diphenyl-1,1,3,3-tetramethyldisilazane are present as starting components A).

4. Catalyst according to at least one of Claims 1 to 3, **characterized in that** potassium fluoride, cesium fluoride and 1,1,1,3,3,3-hexakis(dimethylamino)diphosphazenium fluoride are present as starting components B).

5. Catalyst according to at least one of Claims 1 to 3, **characterized in that** bis(2-methoxyethyl)aminotris-(pyrrolidino)phosphonium fluoride, bis(2-methoxyethyl)aminotris(piperidino)phosphonium fluoride, bis(2-methoxyethyl)aminotris(dimethylamino)phosphonium fluoride, morpholinotris(diethylamino)phosphonium fluoride, bis(2-methoxyethyl)aminosulfur trifluoride, diethylaminosulfur trifluoride, tetrabutylammonium triphenyldifluorosilicate, tetrabutylammonium triphenyldifluorostannate, tetrabutylammonium hexafluorosilicate are present as starting compounds B) .

6. Process for preparing a compound comprising the reaction product of
A) compounds of the general formula I
R_{(4-q)}Si (NR¹R²)_{q} (I),
in which q = 1 or 2,
R simultaneously or independently of one another stands for a saturated or unsaturated, linear or branched aliphatic or cycloaliphatic radical or aryl, aralkyl, or alkylaryl radical having from 1 to 16 carbon atoms, and two radicals R can be linked with one another via an alkylene bridge,
R¹ represents R, SiR₃ or an amide radical of formula (II) 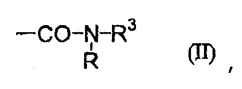 R² is R or H, it being possible for R², if R¹ is not an amide radical, to be linked to R¹ via an alkylene bridge, and
R³ is R or SiR₃,
and
B) compounds selected from alkali metal fluorides, alkaline earth metal fluorides, phosphazenium fluorides, alkylarylaminosulfur trifluorides, dialkylaminosulfur trifluorides, diarylaminosulfur trifluorides, tetraalkylammonium triphenyldifluorosilicates, tetraalkylammonium triphenyldifluorostannates, and tetraalkylammonium hexafluorosilicates, aminophosphonium fluorides of the general formula (III) 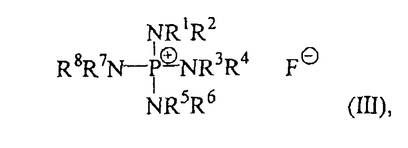
where R¹, R², R³ , R⁴ , R⁵, R⁶, R⁷, and R⁸ are branched or else unbranched aliphatic, optionally alkoxy-substituted alkyl radicals having from 1 to 8 carbon atoms and where in each case the pairings R¹ and R² and also R³ and R⁴ and also R⁵ and R⁶ and also R⁷ and R⁸ may be linked to one another via an alkylene bridge, which where appropriate may also contain the heteroatoms O, S or N,
in a ratio 1/100 < A/B < 100/1,
in the presence if desired of a solvating and/or complexing agent and/or phase transfer catalyst, at a temperature from -20 to 200°C.

7. Process according to Claim 6, **characterized in that** starting compounds according to Claims 2 or 3 and 4 or 5 are used.

8. Use of the compounds according to any of Claims 1 to 6 for trimerizing mono-, di- or polyisocyanates.

9. Use according to Claim 8, **characterized in that** isocyanate compounds used comprise aliphatic, cycloaliphatic, araliphatic and/or aromatic mono-, di- or polyisocyanates.

10. Use according to Claim 9, **characterized in that** cyclohexane diisocyanates, methylcyclohexane diisocyanates, ethylcyclohexane diisocyanates, propylcyclohexane diisocyanates, methyldiethyl-cyclohexane diisocyanates, phenylene diisocyanates, tolylene diisocyanates, bis(isocyanatophenyl)methane, propane diisocyanates, butane diisocyanates, pentane diisocyanates, hexane diisocyanates, such as hexamethylene diisocyanate (HDI) or 1,5-diisocyanato-2-methylpentane (MPDI), heptane diisocyanates, octane diisocyanates, nonane diisocyanates, such as 1,6-diisocyanato-2,4,4-trimethylhexane and 1,6-diisocyanato-2,2,4-trimethylhexane (TMDI), nonane triisocyanates, such as 4-isocyanatomethyl-1,8-octane diisocyanate (TIN), decane di- and triisocyanates, undecane di- and triisocyanates, dodecane di- and triisocyanates, isophorone diisocyanate (IPDI), bis(isocyanatomethylcyclo-hexyl)methane (H₁₂MDI), isocyanatomethyl methylcyclohexyl isocyanates, 2,5(2,6)-bis(iso-cyanatomethyl)bicyclo[2.2.1]heptane (NBDI), 1,3-bis(isocyanatomethyl)cyclohexane (1,3-H₆-XDI), or 1,4-bis(isocyanatomethyl)cyclohexane (1,4-H₆-XDI) are used.

11. Use according to Claim 9, **characterized in that** HDI, IPDI, MPDI, TMDI, 1,3- and 1,4-H₆-XDI, NBDI or mixtures of HDI and IPDI are used.

12. The use according to Claim 9, **characterized in that** monoisocyanates used comprise ethyl isocyanate, cyclohexyl isocyanate, phenyl isocyanate, tolyl isocyanate, benzyl isocyanate, and all regioisomers and stereoisomers of the following compounds: propyl isocyanates, hexyl isocyanates, octyl isocyanates, and methoxypropyl isocyanate.

13. Process for preparing polyisocyanates containing isocyanurate groups by catalytically induced trimerization of organic mono-, di- or polyisocyanates, **characterized in that** trimerization catalysts comprising the reaction product of
A) compounds of the general formula I
R_{(4-q)}Si (NR¹R²)_{q} (I),
in which q = 1 or 2,
R simultaneously or independently of one another represents a saturated or unsaturated, linear or branched aliphatic or cycloaliphatic radical or aryl, aralkyl, or alkylaryl radical having from 1 to 16 carbon atoms, and two radicals R can be linked with one another via an alkylene bridge,
R¹ represents R, SiR₃ or an amide radical of formula (II) 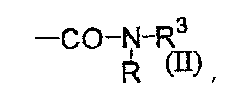 R² is R or H, it being possible for R², if R¹ is not an amide radical, to be linked to R¹ via an alkylene bridge, and
R³ is R or SiR₃,
and
B) compounds selected from alkali metal fluorides, alkaline earth metal fluorides, phosphazenium fluorides, alkylarylaminosulfur trifluorides, dialkylaminosulfur trifluorides, diarylaminosulfur trifluorides, tetraalkylammonium triphenyldifluorosilicates, tetraalkylammonium triphenyldifluorostannates, and tetraalkylammonium hexafluorosilicates, aminophosphonium fluorides of the general formula (III) 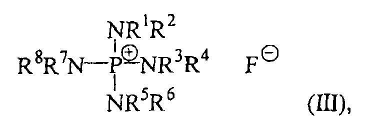
where R¹, R², R³, R⁴ , R⁵, R⁶, R⁷, and R⁸ are branched or else unbranched aliphatic, optionally alkoxy-substituted alkyl radicals having from 1 to 8 carbon atoms and where in each case the pairings R¹ and R² and also R³ and R⁴ and also R⁵ and R⁶ and also R⁷ and R⁸ may be linked to one another via an alkylene bridge, which where appropriate may also contain the heteroatoms O, S or N,
are used in a ratio 1/100 < A/B < 100/1.

14. Process according to Claim 13, **characterized in that** aliphatic, cycloaliphatic, araliphatic or aromatic mono-, di- or polyisocyanates are used.

15. Process according to Claim 14, **characterized in that** cyclohexane diisocyanates, methylcyclohexane diisocyanates, ethylcyclohexane diisocyanates, propylcyclohexane diisocyanates, methyldiethyl-cyclohexane diisocyanates, phenylene diisocyanates, tolylene diisocyanates, bis(isocyanatophenyl)methane, propane diisocyanates, butane diisocyanates, pentane diisocyanates, hexane diisocyanates, such as hexamethylene diisocyanate (HDI) or 1,5-diisocyanato-2-methylpentane (MPDI), heptane diisocyanates, octane diisocyanates, nonane diisocyanates, such as 1,6-diisocyanato-2,4,4-trimethylhexane and 1,6-diisocyanato-2,2,4-trimethylhexane (TMDI), nonane triisocyanates, such as 4-isocyanatomethyl-1,8-octane diisocyanate (TIN), decane di- and triisocyanates, undecane di- and triisocyanates, dodecane di- and triisocyanates, isophorone diisocyanate (IPDI), bis(isocyanatomethylcyclo-hexyl)methane (H₁₂MDI), isocyanatomethyl methylcyclohexyl isocyanates, 2,5(2,6)-bis(iso-cyanatomethyl)bicyclo[2.2.1]heptane (NBDI), 1,3-bis(isocyanatomethyl)cyclohexane (1,3-H₆-XDI), or 1,4-bis(isocyanatomethyl)cyclohexane (1,4-H₆-XDI) are used.

16. Process according to Claim 14, **characterized in that** HDI, IPDI, MPDI, TMDI, 1,3- and 1,4-H₆-XDI, NBDI or mixtures of HDI and IPDI are used.

17. Process according to Claim 14, **characterized in that** monoisocyanates used comprise ethyl isocyanate, cyclohexyl isocyanate, phenyl isocyanate, tolyl isocyanate, benzyl isocyanate, and all regioisomers and stereoisomers of the following compounds: propyl isocyanates, hexyl isocyanates, octyl isocyanates, and methoxypropyl isocyanate.

18. Process according to at least one of Claims 13 to 17, **characterized in that** the preparation of the polyisocyanates containing isocyanurate groups is conducted continuously or batchwise.

19. Process according to at least one of Claims 13 to 18, **characterized in that** the catalysts are used at a concentration of between 0.01 and 5.0% by weight.

20. Process according to at least one of Claims 13 to 19, **characterized in that** the preparation is carried out isothermally in a temperature range between 0°C and 100°C, preferably between 20°C and 80°C.

21. Use of the monomer-free, isocyanurate-functional polyisocyanates prepared according to at least one of Claims 13 to 20 as intermediates for polyurethane coatings, polyurethane dispersions, adhesives, and as a polyisocyanate component in 1- and 2-component polyurethane systems.

22. Use according to Claim 21 for leather coatings and textile coatings or polyurethane coating materials.

## Revendications

1. Catalyseur pour effectuer la trimérisation d'isocyanates, constitué du produit de la réaction effectuée entre :
A) des composés de formule générale I
R_{(4-q)}Si(NR¹R²)_{q} (I),
dans laquelle q = 1 ou 2,
les radicaux R représentent, simultanément ou indépendamment les uns des autres, un radical aliphatique ou cycloaliphatique, linéaire ou ramifié, saturé ou insaturé, ou un radical aryle, aralkyle ou alkylaryle ayant 1 à 16 atomes de carbone, et deux radicaux R peuvent être combinés l'un avec l'autre par le biais d'un pont alkylène,
R¹ représente R, SiR₃ ou un radical amide de formule (II) 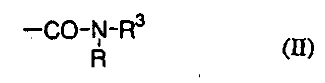 R² est identique à R ou représente H, et - si R¹ n'est pas un radical amide - R² peut être combiné avec R¹ par le biais d'un pont alkylène, et
R³ est identique à R ou représente SiR₃,
et
B) des composés choisis parmi les fluorures de métaux alcalins, les fluorures de métaux alcalinoterreux, les fluorures de phosphazénium, les trifluorures d'alkylarylaminosoufre, les trifluorures de dialkylaminosoufre, les trifluorures de diarylaminosoufre, les triphényldifluorosilicates de tétraalkylammonium, les triphényldifluorostannates de tétraalkylammonium et les hexafluorosilicates de tétraalkylammonium, les fluorures d'aminophosphonium de formule générale (III) 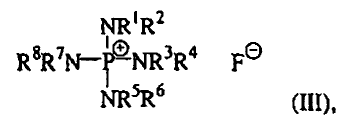
dans laquelle R¹, R², R³, R⁴, R⁵, R⁶, R⁷ et R⁸ représentent des radicaux alkyle aliphatiques, ramifiés ou non ramifiés, éventuellement substitués par un alcoxy, ayant 1 à 8 atomes de carbone, chaque paire R¹ et R², R³ et R⁴, R⁵ et R⁶ ainsi que R⁷ et R⁸, pouvant être combinée par le biais d'un pont alkylène, lequel peut, éventuellement, également contenir les hétéroatomes O, S ou N,
dans le rapport 1/100 < A/B < 100/1.

2. Catalyseur selon la revendication 1, **caractérisé en ce qu'**il contient des aminosilanes, des silylurées et/ou des silazanes comme composants de départ A).

3. Catalyseur selon au moins l'une quelconque des revendications 1 et 2, **caractérisé en ce qu'**il contient comme composants de départ A) les composés suivants: méthylaminotriméthylsilane, diméthylaminotriméthylsilane, dibutylaminotriméthylsilane, diéthylaminodiméthylphénylsilane, bis(diméthylamino)diméthylsilane, bis(diéthylamino)diméthylsilane, bis(dibutylamino)diméthylsilane, bis(diméthylamino)méthylphénylsilane, N-méthyl-N-triméthylsilyl-N'-méthyl-N'-butylurée, N-triméthylsilyl-N-méthyl-N',N'-diméthylurée, N-triméthylsilyl-N-éthyl-N',N'-diméthylurée, N-triméthylsilyl-N-butyl-N'-butyl-N'-triméthylsilylurée, triméthylsilylpyrrolidine, triméthylsilylmorpholine, triméthylsilylpipéridine, triméthylsilylpipérazine, hexaméthyldisilazane, heptaméthyldisilazane, 1,3-diéthyl-1,1,3,3-tétraméthyldisilazane, hexaéthyldisilazane et/ou 1,3-diphényl-1,1,3,3-tétraméthyldisilazane.

4. Catalyseur selon au moins l'une quelconque des revendications 1 à 3, **caractérisé en ce qu'**il contient du fluorure de potassium, du fluorure de césium, du fluorure de 1,1,1,3,3,3-hexakis-(diméthylamino)diphosphazénium comme composants de départ B).

5. Catalyseur selon au moins l'une quelconque des revendications 1 à 3, **caractérisé en ce qu'**il contient comme composants de départ B) les composés suivants: fluorure de bis (2-méthoxyéthyl)aminotris(pyrrolidino)phosphonium, fluorure de bis(2-méthoxyéthyl)amino-tris(pipéridino)phosphonium, fluorure de bis(2-méthoxyéthyl)aminotris(diméthylamino)phosphonium, fluorure de morpholino-tris(diéthylamino)phosphonium, trifluorure de bis(2-méthoxyéthyl)aminosoufre, trifluorure de diéthylaminosoufre, triphényldifluorosilicate de tétrabutylammonium, triphényldifluorstannate de tétrabutylammonium, hexafluorosilicate de tétrabutylammonium.

6. Procédé de fabrication d'un composé constitué du produit de la réaction effectuée entre :
A) des composés de formule générale I
R_{(4-q)}Si(NR¹R²)_{q} (I),
dans laquelle q = 1 ou 2,
les radicaux R représentent, simultanément ou indépendamment les uns des autres, un radical aliphatique ou cycloaliphatique, linéaire ou ramifié, saturé ou insaturé, ou un radical aryle, aralkyle ou alkylaryle ayant 1 à 16 atomes de carbone, et deux radicaux R peuvent être combinés l'un avec l'autre par le biais d'un pont alkylène,
R¹ représente R, SiR₃ ou un radical amide de formule (II) 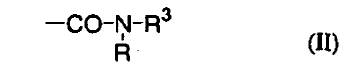 R² est identique à R ou représente H, et - si R¹ n'est pas un radical amide - R² peut être combiné avec R¹ par le biais d'un pont alkylène, et
R³ est identique à R ou représente SiR₃,
et
B) des composés choisis parmi les fluorures de métaux alcalins, les fluorures de métaux alcalinoterreux, les fluorures de phosphazénium, les trifluorures d'alkylarylaminosoufre, les trifluorures de dialkylaminosoufre, les trifluorures de diarylaminosoufre, les triphényldifluorosilicates de tétraalkylammonium, les triphényldifluorostannates de tétraalkylammonium et les hexafluorosilicates de tétraalkylammonium, les fluorures d'aminophosphonium de formule générale (III) 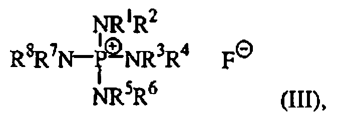
dans laquelle R¹, R², R³, R⁴, R⁵, R⁶, R⁷ et R⁸ représentent des radicaux alkyle aliphatiques, ramifiés ou non ramifiés, éventuellement substitués par un alcoxy, ayant 1 à 8 atomes de carbone, chaque paire R¹ et R², R³ et R⁴, R⁵ et R⁶ ainsi que R⁷ et R⁸ pouvant être combinée par le biais d'un pont alkylène, lequel peut, éventuellement, également contenir les hétéroatomes O, S ou N,
dans le rapport 1/100 < A/B < 100/1,
éventuellement en présence d'un agent de solvatation et/ou d'un agent complexant et/ou d'un catalyseur à transfert de phase, à une température de -20 °C à 200°C.

7. Procédé selon la revendication 6, **caractérisé en ce que** l'on utilise des composés de départ selon les revendications 2 ou 3 et 4 ou 5.

8. Utilisation des composés selon l'une quelconque des revendications 1 à 6, pour effectuer la trimérisation de monoisocyanates, diisocyanates ou polyisocyanates.

9. Utilisation selon la revendication 8, **caractérisée en ce que** l'on utilise, comme composés d'isocyanate, des monoisocyanates, diisocyanates ou polyisocyanates aliphatiques, cycloaliphatiques, araliphatiques et/ou aromatiques.

10. Utilisation selon la revendication 9, **caractérisée en ce que** l'on utilise les composés suivants : diisocyanates de cyclohexane, diisocyanates de méthylcyclohexane, diisocyanates d'éthylcyclohexane, diisocyanates de propylcyclohexane, diisocyanates de méthyldiéthylcyclohexane, diisocyanates de phénylène, diisocyanates de toluylène, bis(isocyanatophényl)méthane, diisocyanates de propane, diisocyanates de butane, diisocyanates de pentane, diisocyanates d'hexane, tels que le diisocyanate d'hexaméthylène (HDI) ou le 1,5-diisocyanato-2-méthylpentane (MPDI), diisocyanates d'heptane, diisocyanates d'octane, diisocyanates de nonane, tels que le 1,6-diisocyanato-2,4,4-triméthylhexane et le 1,6-diisocyanato-2,2,4-triméthylhexane (TMDI), triisocyanates de nonane, tels que le 4-isocyanatométhyl-1,8-octanediisocyanate (TIN), diisocyanates et triisocyanates de décane, diisocyanates et triisocyanates d'undécane, diisocyanates et triisocyanates de dodécane, diisocyanate d'isophorone (IPDI), bis(isocyanatométhylcyclohexyl)méthane (H₁₂MDI), isocyanatométhyl-méthylcyclohexylisocyanate, 2,5(2,6)-bis-(isocyanatométhyl)bicyclo[2.2.1]heptane (NBDI), 1,3-bis(isocyanatométhyl)cyclohexane (1,3-H₆-XDI) ou 1,4-bis(isocyanatométhyl)cyclohexane (1,4-H₆-XDI).

11. Utilisation selon la revendication 9, **caractérisée en ce que** l'on utilise les composés HDI, IPDI, MPDI, TMDI, 1,3- et 1,4-H₆-XDI, NBDI ou des mélanges de HDI et d'IPDI.

12. Utilisation selon la revendication 9, **caractérisée en ce que** l'on utilise, comme monoisocyanates, les composés suivants : isocyanate d'éthyle, isocyanate de cyclohexyle, isocyanate de phényle, isocyanate de tolyle, isocyanate de benzyle, ainsi que tous les régioisomères et stéréoisomères des composés suivants : isocyanates de propyle, isocyanates d'hexyle, isocyanates d'octyle et isocyanates de méthoxypropyle.

13. Procédé de fabrication de polyisocyanates contenant des groupements isocyanurate, par trimérisation induite par voie catalytique de monoisocyanates, diisocyanates et polyisocyanates organiques, **caractérisé en ce que** l'on utilise des catalyseurs de trimérisation constitués du produit de la réaction entre:
A) des composés de formule générale I
R_{(4-q)}Si(NR¹R²)_{q} (I),
dans laquelle q = 1 ou 2,
les radicaux R représentent, simultanément ou indépendamment les uns des autres, un radical aliphatique ou cycloaliphatique, linéaire ou ramifié, saturé ou insaturé, ou un radical aryle, aralkyle ou alkylaryle ayant 1 à 16 atomes de carbone, et deux radicaux R peuvent être combinés l'un avec l'autre par le biais d'un pont alkylène,
R¹ représente R, SiR₃ ou un radical amide de formule (II) 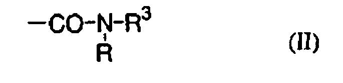 R² est identique à R ou représente H, et - si R¹ n'est pas un radical amide - R² peut être combiné avec R¹ par le biais d'un pont alkylène, et
R³ est identique à R ou représente SiR₃,
et
B) des composés choisis parmi les fluorures de métaux alcalins, les fluorures de métaux alcalinoterreux, les fluorures de phosphazénium, les trifluorures d'alkylarylaminosoufre, les trifluorures de dialkylaminosoufre, les trifluorures de diarylaminosoufre, les triphényldifluorosilicates de tétraalkylammonium, les triphényldifluorostannates de tétraalkylammonium et les hexafluorosilicates de tétraalkylammonium, les fluorures d'aminophosphonium de formule générale (III) 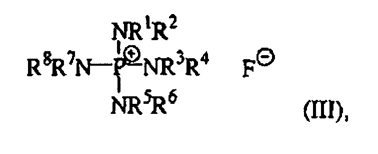
dans laquelle R¹, R², R³, R⁴, R⁵, R⁶, R⁷ et R⁸ représentent des radicaux alkyle aliphatiques, ramifiés ou non ramifiés, éventuellement substitués par un alcoxy, ayant 1 à 8 atomes de carbone, chaque paire R¹ et R², R³ et R⁴, R⁵ et R⁶ ainsi que R⁷ et R⁸ pouvant être combinée par le biais d'un pont alkylène, lequel peut, éventuellement, également contenir les hétéroatomes O, S ou N,
dans le rapport 1/100 < A/B < 100/1.

14. Procédé selon la revendication 13, **caractérisé en ce que** l'on utilise des monoisocyanates, diisocyanates ou polyisocyanates aliphatiques, cycloaliphatiques, araliphatiques ou aromatiques.

15. Procédé selon la revendication 14, **caractérisé en ce que** l'on utilise les composés suivants : diisocyanates de cyclohexane, diisocyanates de méthylcyclohexane, diisocyanates d'éthyl-cyclohexane, diisocyanates de propylcyclohexane, diisocyanates de méthyldiéthylcyclohexane, diisocyanates de phénylène, diisocyanates de toluylène, bis(isocyanatophényl)méthane, diisocyanates de propane, diisocyanates de butane, diisocyanates de pentane, diisocyanates d'hexane, tels que le diisocyanate d'hexaméthylène (HDI) ou le 1,5-diisocyanato-2-méthylpentane (MPDI), diisocyanates d'heptane, diisocyanates d'octane, diisocyanates de nonane, tels que le 1,6-diisocyanato-2,4,4-triméthylhexane et le 1,6-diisocyanato-2,2,4-triméthylhexane (TMDI), triisocyanates de nonane, tels que le 4-isocyanatométhyl-1,8-octanediisocyanate (TIN), diisocyanates et triisocyanates de décane, diisocyanates et triisocyanates d'undécane, diisocyanates et triisocyanates de dodécane, diisocyanate d'isophorone (IPDI), bis(isocyanatométhylcyclohexyl)méthane (H₁₂MDI), isocyanatométhyl-méthylcyclohexylisocyanate, 2,5(2,6)-bis-(isocyanatométhyl)bicyclo[2.2.1]heptane (NBDI), 1,3-bis(isocyanatométhyl)cyclohexane (1,3-H₆-XDI) ou 1,4-bis(isocyanatométhyl) cyclohexane (1,4-H₆-XDI).

16. Procédé selon la revendication 14, **caractérisé en ce que** l'on utilise les composés HDI, IPDI, MPDI, TMDI, 1,3- et 1,4-H₆-XDI, NBDI ou des mélanges de HDI et d'IPDI.

17. Procédé selon la revendication 14, **caractérisé en ce que** l'on utilise des monoisocyanates choisis parmi les composés suivants : isocyanate d'éthyle, isocyanate de cyclohexyle, isocyanate de phényle, isocyanate de tolyle, isocyanate de benzyle, ainsi que tous les régioisomères et stéréoisomères des composés suivants : isocyanates de propyle, isocyanates d'hexyle, isocyanates d'octyle et isocyanates de méthoxypropyle.

18. Procédé selon au moins l'une quelconque des revendications 13 à 17, **caractérisé en ce que** la fabrication des polyisocyanates contenant des groupements isocyanurate est réalisée en continu ou en discontinu.

19. Procédé selon au moins l'une quelconque des revendications 13 à 18, **caractérisé en ce que** les catalyseurs sont utilisés en une concentration comprise entre 0,01 et 5,0 % en poids.

20. Procédé selon au moins l'une quelconque des revendications 13 à 19, **caractérisé en ce que** la fabrication est réalisée en conditions isothermiques, dans une plage de températures comprises entre 0 °C et 100 °C, de préférence entre 20 °C et 80 °C.

21. Utilisation des polyisocyanates contenant des groupements isocyanurate, exempts de monomères, fabriqués selon au moins l'une quelconque des revendications 13 à 20, comme produits intermédiaires pour des revêtements de polyuréthanne, des dispersions de polyuréthanne, des colles et comme composés de polyisocyanate dans des systèmes de polyuréthanne à 1 ou 2 composés.

22. Utilisation selon la revendication 21, pour le revêtement du cuir et des textiles ou pour les vernis de polyuréthanne.
